# EUROPEAN PATENT APPLICATION

(11) **EP 4 763 092 A1**
(43) Date of publication of application: **24.06.2026**
(21) Application number: 24220579.7
(22) Date of filing: 17.12.2024
(51) Int. Cl.: A61B 6/58, A61B 6/08

(54) **X-RAY IMAGING SYSTEM**

(71) Applicant: Koninklijke Philips N.V., 5656 AE Eindhoven (NL)
(72) Inventor: BYSTROV, Daniel, Eindhoven (NL); GOOSSEN, André, Eindhoven (NL); YOUNG, Stewart Matthew, 5656AG Eindhoven (NL); SENEGAS, Julien Thomas, Eindhoven (NL); KROENKE-HILLE, Sven, Eindhoven (NL); VON BERG, Jens, Eindhoven (NL)
(74) Representative: Philips Intellectual Property & Standards

(57) **Abstract**

An X-ray imaging system has a visible light source and associated optics for directing the visible light source output to an X-ray collimator, in order to generate a collimation light field. A 2D camera has an offset between an optical axis of the 2D camera and an optical axis of the X-ray source. The camera is for capturing reflected images formed from the visible light source output, so that a depth of an object between the X-ray source and the X-ray detector can be determined from the 2D camera images based on a parallax shift.

## Description

### FIELD OF THE INVENTION

This invention relates to X-ray imaging systems, and in particular systems which incorporate depth measurement.

### BACKGROUND OF THE INVENTION

A digital radiography X-ray imaging system typically comprises an X-ray tube and an X-ray detector between which a body part to be imaged is positioned. The X-ray detector may for example be integrated into a wall stand, and the patient stands or sits between the X-ray tube and the detector, for example for a chest X-ray. The X-ray detector may instead be a mobile detector panel. It may for example be placed on a flat surface with the body part (e.g., a limb) on top, with the X-ray tube positioned above.

For the automation and workflow optimization in X-ray imaging, an estimation of the distance between the X-ray source and the target body part of the patient is of interest.

For example, it is known to incorporate a depth camera into the system. It is known to use the obtained depth information to assist in accurately positioning the patient, for example by capturing a 3D contour of their body. This information can be used to ensure that the body part being imaged is in the correct orientation and distance relative to the X-ray source and detector, minimizing errors caused by misalignment. This helps to reduce the need for repeat scans due to incorrect positioning.

By analyzing the patient's body contours, a depth camera can also help adjust X-ray parameters (such as intensity) based on the thickness or depth of the area being imaged.

Depth cameras can also support automation in digital radiography by integrating with imaging software. For example, by identifying the patient's body position the X-ray system may be adjusted automatically. Depth cameras can also track patient movement in real-time, ensuring that the patient remains in the correct position throughout the imaging process.

The use of a depth camera within a digital radiography system is for example disclosed in US 2014/0355735. However, this adds complex hardware to the imaging system.

There is a need for a simpler way to measure the distance between the X-ray source and the object being imaged.

### SUMMARY OF THE INVENTION

The invention is defined by the claims.

According to examples in accordance with an aspect of the invention, there is provided an X-ray imaging system, comprising:
an X-ray source;
an X-ray collimator for generating an X-ray field from the output of the X-ray source;
an X-ray detector;
a visible light source and optics for directing the visible light source output to the X-ray collimator, in order to generate a collimation light field;
a 2D camera with an offset between the optical axis of the 2D camera and the optical axis of the X-ray source for capturing reflected images formed from at least the visible light source output; and
a processor for processing images to determine a depth of an object between the X-ray source and the X-ray detector based on a parallax shift.

This system determines a depth of an object (i.e., a body portion that is to be imaged) using a 2D camera. This avoids the need for a depth camera. The 2D camera is able to derive a depth value, or more preferably a depth map, by using the collimation light field, and in particular with an offset between the camera optical axis and the central axis of the collimation light field (which is aligned with the collimated X-ray beam). This offset creates a parallax in the captured image when the captured image has different depth portions. In this way, a calibrated standard 2D camera that is close to the optical axis of the X-ray beam is used to image the existing collimation light field of the imaging system. This collimation light field is conventionally used for visual positioning purposes.

The reflected images that are formed from the visible light source output (and other available light in the acquisition room) are reflected off the patient or reflected off the detector (e.g., a wall stand or a mobile detector panel).

The invention thereby solves the problem of estimating the distance between the X-ray source and a target anatomy without the burden of integrating and calibrating a depth camera in the tube head of the X-ray system, which causes technical (thermal) as well as supply chain problems.

The 2D camera is for example mounted between the collimator and X-ray detector. It may be mounted on the outside of the collimator housing.

The system may further comprise a pattern generator for generating a patterned collimation light field. This creates a pattern that is more suitable for depth analysis, for example with a dense arrangement of lines.

The patterned collimation light field for example comprises a grid pattern.

The optics for example comprises a wavelength-dependent mirror.

The invention also provides a method of determining a depth of an object between an X-ray source and an X-ray detector of an X-ray imaging system, comprising;
generating a collimation light field by providing a visible light source output to an X-ray collimator;
capturing reflected images formed from the visible light source output using a 2D camera with an offset between an optical axis of the 2D camera and an optical axis of the X-ray source; and
processing the images to determine a depth of an object between the X-ray source and the X-ray detector based on a parallax shift.

The method may further comprise generating a patterned collimation light field. The patterned collimation light field for example comprises a grid pattern.

The invention also provides a computer program comprising computer program code that is adapted, when said program is run on a computer to perform a method comprising:
receiving 2D camera images of a visible light collimation light field;
processing the images to determine a depth of an object between an X-ray source and an X-ray detector based on a parallax shift resulting from an offset between an optical axis of the 2D camera and an optical axis of the X-ray source.

The invention also provides a processor, having stored thereon the computer program described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an X-ray imaging system;
Fig. 2 shows a captured image using optical collimation light;
Fig. 3 shows an alternative collimation light pattern; and
Fig. 4 shows a method of determining a distance from an X-ray source to an object.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an X-ray imaging system having a visible light source and associated optics for directing the visible light source output to an X-ray collimator, in order to generate a collimation light field. A 2D camera is provided with an optical axis with an offset to the optical axis of the X-ray source. The camera is for capturing reflected images formed from the visible light source output (and the available light in the acquisition room), so that a depth of an object between the X-ray source and the X-ray detector can be determined from the 2D camera images based on a parallax shift.

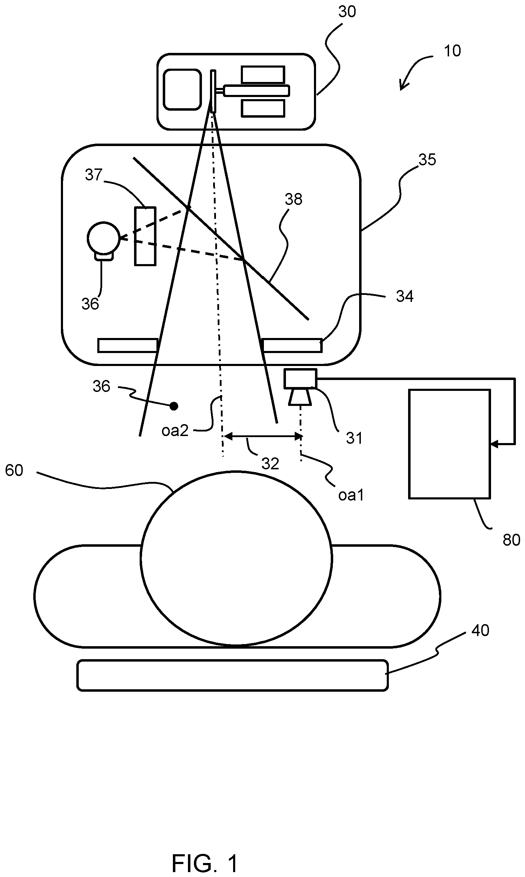

Fig. 1 shows an X-ray imaging system 10, comprising an X-ray source 30 and an X-ray detector 40, between which a body part of a subject 60 is positioned for X-ray imaging.

In the example shown, the view of Fig. 1 is from above, so the subject is standing and the X-ray detector 40 is integrated into a wall stand. This is a typical layout for a chest X-ray. The detector 40 may instead be part of a mobile panel, and a body part such as a hand or foot is placed over the detector, with the X-ray source positioned above.

The X-ray source 30 comprises an X-ray tube, and an X-ray collimator 34 is provided at the output of the X-ray source 30, for generating an X-ray field 36 from the output of the X-ray source 30. The collimator 34 is within a collimator housing 35.

Collimators are used in X-ray detectors to restrict the angles at which X-rays can reach the detector 40. This process reduces the delivered X-ray dose to the patient and also minimizes background noise and improves image quality by filtering out scattered rays that can distort the signal. The collimator restricts the X-ray beam to a particular width and height before it reaches the detector, and the collimated area is essentially the footprint of that beam on the detector's surface.

A visible light source 36 is provided together with optics 38 in the form of a wavelength-selective mirror for directing the visible light source output to the X-ray collimator 34, in order to generate a collimation light field formed of visible light (i.e., in the visible light spectrum). The visible light source is provided, in this example, in the collimator housing 35. Instead of a wavelength-selective mirror, a removable mirror may be used that is in place for the optical analysis and is removed for the X-ray exposure.

The use of a visible collimation light field is well-known, for example for visually aligning the detector with the irradiation area of the X-ray source. For this purpose, the collimation light field aligns with the X-ray field 36. This visualization is used to align and check the coverage area without directly exposing the area to X-rays, which can be invisible and difficult to measure visually. Thus, collimation light is primarily used for alignment, positioning, and ensuring that the X-ray beam will cover the intended area of the patient and the detector.

The visible light source for example comprises a light bulb or laser. The light beam is adjusted to match the X-ray field by using a mirror (as shown), or multiple mirrors and/or lenses that direct it through the same aperture as the X-rays. The light is thus focused to create a narrow, directed beam that mimics the path of the X-ray beam through the collimator's opening. In this way, the light forms an outline of the area that will be irradiated on the detector. For example, it is known to use collimation light to project a visible rectangle, square, or circle (depending on the collimator shape) on the detector or the subject. This projection visually represents the boundaries of the X-ray field.

An operator of the X-ray imaging system may for example adjust the collimator to change the field size of the collimation light beam, which translates to adjusting the X-ray field size.

As discussed above, it is known to generate a depth value or depth map of the patient, to assist in workflow planning and implementation. The depth map reports the distance between the patient and the X-ray source.

For generating a depth map in accordance with the teachings of this disclosure, Fig. 1 shows a 2D camera 31 for capturing the collimation light field after reflection from the patient or detector.

It is noted that the use of a camera is known, to provide additional information to the radiographer. Thus, the radiographer may perform visual inspection of both an X-ray image and a camera image of the patient in order to assist in the interpretation of the X-ray image.

This disclosure is based on the use of the camera for depth analysis. For this purpose, there is an offset 32 between an optical axis oa1 of the 2D camera 31 and an optical axis oa2 of the X-ray source 30 (which corresponds to the optical axis of the collimation light field).

The optical axes oa1 and oa2 may be parallel so that the offset is a spacing between the optical axes. However, the offset may instead comprise an angular offset between the optical axes.

Byway of example, a spatial offset may be in the range 1cm to 10cm, and/or an angular offset may be in the range 1 to 10 degrees.

The 2D camera 31 is for example mounted outside the collimator housing 35 facing the detector. The effect of the offset 32 is to create a parallax between the collimation light field and the camera field of view.

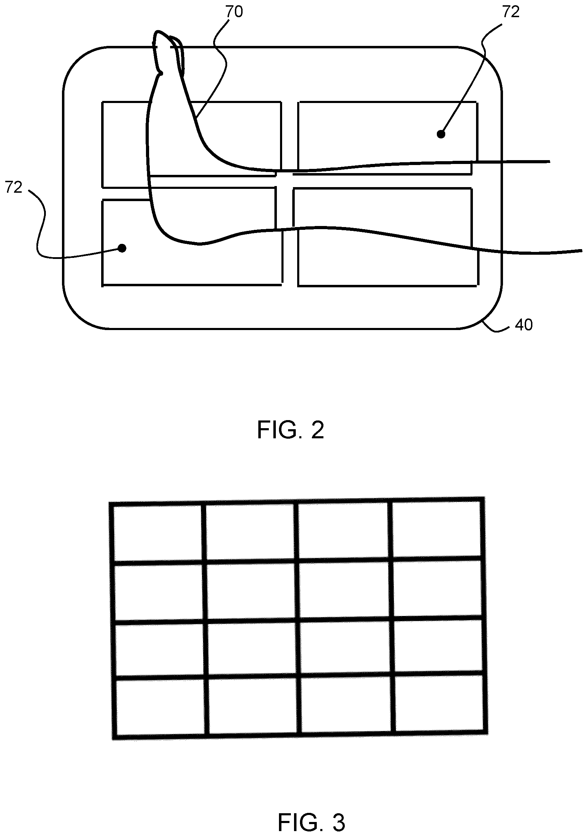

Fig. 2 shows the effect of this parallax.

It shows the shape of the reflected collimation light field, when the original light field generated by the visible light source comprises a pattern of four illuminated rectangles 72.

The collimation light field is reflected from the detector 40 where the body part does not obstruct the view of the detector, and is reflected from the body part where the body part does obstruct the view of the detector 40.

A foot 70 is shown over the detector 40. The parallax means that straight edges are distorted when they are reflected back from different depths, as shown.

From the visible parallax offset in the captured camera image, the depth of the patient from the X-ray source, where the patient is obstructing the view of the detector, can be calculated, and a depth value can thereby be derived.

For this purpose, a processor 80 (in Fig. 1) is provided for processing the captured images to determine a depth of an object between the X-ray source and the X-ray detector based on the parallax shift. A well-known structured light algorithm may be used to compute the local distances to the camera, which is calibrated to the collimation light source.

For example, structured light depth imaging is explained in the reference book "Time-of-Flight and Structured Light Depth Cameras", Pietro Zanuttigh, Giulio Marin, Carlo Dal Mutto, Fabio Dominio, Ludovico Minto, Guido Maria Cortelazzo, Springer Cham, 2018.

The approach here avoids the need for a depth camera. The 2D camera is able to derive a depth value or depth map by using the collimation light field, and in particular using the offset between the camera optical axis and the collimation light (which is aligned with the collimated X-ray). This offset creates a parallax in the captured image, when the captured image has different depth portions. In this way, a calibrated standard 2D camera that is close to the optical axis of the X-ray beam is used to image the existing collimation light field of the imaging system.

The camera is calibrated so that the image of the detector (with no body part present) acts as a reference. Deviations from this reference result from reflections before the detector. The collimation light field, in particular the edges, may be used for this calibration of the 2D camera with the X-ray system. This for example may assist in the installation of an external camera.

A most basic collimation light field may simply comprise a rectangle. This may for example enable the depth at the corners of the rectangle to be assessed. The corners are identifiable locations of the collimation light field, and thus their locations can be compared to the reference.

A more structured light field allows more depth values to be determined. For example, a grid of four (2x2) rectangular illuminations may enable the depth at all 16 rectangle corners to be determined. Thus, the pattern may comprise a set of crosshairs defining rectangular areas between the crosshairs, and the central beam in the middle.

There may for example be nine (3x3) rectangular illumination areas defined by a pattern of four crosshairs.

An improved light pattern may be generated, for use as the collimation light field, as shown in Fig. 3. This pattern has six crosshairs to form sixteen (4x4) illumination areas. The more complex grid pattern improves the resolution of the depth map.

A pattern generator 37 (shown in Fig. 1) may be used at the output of the visible light source for generating the patterned collimation light field such as shown in Figs. 2 and 3.

In addition to the spatial pattern of the collimation light field, the intensity of the collimation light field may be modulated in the collimated area. For example, it is known to apply spatial patterns for depth imaging, comprising stripes of varying brightness, gradients or dots with different intensities.

The possible uses of the depth map will depend on the resolution of the depth image. The detected location of the body may be used to adjust the position of a wall stand detector. A higher resolution depth map may enable the position and orientation of body parts to be identified automatically, so that suggested positional adjustment of the body part (e.g., a foot/ankle as shown in Fig. 2) may be generated.

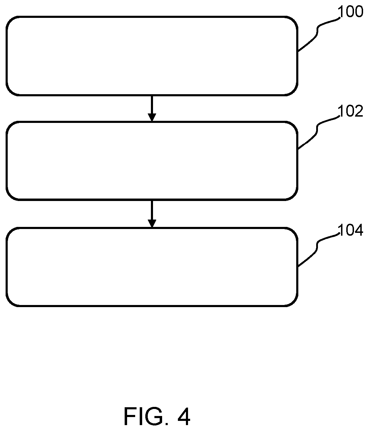

Fig. 4 shows a method of determining a depth of an object between an X-ray source and an X-ray detector of an X-ray imaging system. The method comprises:
In step 100, a collimation light field is generated by providing a visible light source output to an X-ray collimator.
In step 102, images of the visible light source output are captured using a 2D camera with an offset between an optical axis of the 2D camera and an optical axis of the X-ray source.
In step 104, the images are processed to determine a depth of an object between the X-ray source and the X-ray detector based on a parallax shift.

The image processing is performed by software. Thus, a computer program may be provided to perform a method comprising receiving 2D camera images of a visible light collimation light field and processing the images to determine a depth of an object between an X-ray source and an X-ray detector based on a parallax shift resulting from an offset between an optical axis of the 2D camera and an optical axis of the X-ray source.

As discussed above, the system makes use of processor to perform the image processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

The skilled person would be readily capable of developing a processor for carrying out the image processing.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An X-ray imaging system (10), comprising:
an X-ray source (30);
an X-ray collimator (34) for generating an X-ray field (36) from the output of the X-ray source;
an X-ray detector (40);
a visible light source (36) and optics (38) for directing the visible light source output to the X-ray collimator (34), in order to generate a collimation light field;
a 2D camera (31) with a offset (32) between the optical axis (oa1) of the 2D camera (31) and the optical axis (oa2) of the X-ray source (30) for capturing reflected images formed from the visible light source output; and
a processor (80) for processing images to determine a depth of an object (60) between the X-ray source and the X-ray detector based on a parallax shift.

2. The system of claim 1, wherein the 2D camera (31) is mounted between the collimator (34) and X-ray detector (40).

3. The system of claim 1 or 2, further comprising a pattern generator (37) for generating a patterned collimation light field.

4. The system of claim 3, wherein the patterned collimation light field comprises a grid pattern.

5. The system of any one of claims 1 to 4, wherein the optics (38) comprises a wavelength dependent mirror.

6. The system of any one of claims 1 to 4, wherein the optics (38) comprises a removable mirror.

7. The system of any one of claims 1 to 6, wherein the optical axis (oa1) of the 2D camera and the optical axis (oa2) of the X-ray source are parallel and the offset comprises a spacing between them.

8. A method of determining a depth of an object (60) between an X-ray source and an X-ray detector of an X-ray imaging system, comprising;
(100) generating a collimation light field by providing a visible light source output to an X-ray collimator;
(102) capturing reflected images formed from the visible light source output using a 2D camera with an offset (32) between an optical axis (oa1) of the 2D camera and an optical axis (oa2) of the X-ray source; and
(104) processing the images to determine a depth of the object (60) between the X-ray source and the X-ray detector based on a parallax shift.

9. The method of claim 8, further comprising generating a patterned collimation light field.

10. The method of claim 9, wherein the patterned collimation light field comprises a grid pattern.

11. The method of any one of claims 8 to 10, wherein the optical axis (oa1) of the 2D camera and the optical axis (oa2) of the X-ray source are parallel and the offset comprises a spacing between them.

12. A computer program comprising computer program code that is adapted, when said program is run on a computer to perform a method comprising:
receiving 2D camera images of a visible light collimation light field;
processing the images to determine a depth of an object between an X-ray source and an X-ray detector based on a parallax shift resulting from an offset between an optical axis (oa1) of the 2D camera and an optical axis (oa2) of the X-ray source.

13. A processor having stored there on the computer program of claim 12.
